# EUROPEAN PATENT SPECIFICATION

(11) **EP 2 862 509 B1**
(45) Date of publication and mention of the grant of the patent: **06.01.2021**
(21) Application number: 13188896.8
(22) Date of filing: 16.10.2013
(51) Int. Cl.: A61B 5/024, A61B 5/16, A61B 5/0452

(54) **Psychiatric symptom and psychiatric disorder onset risk evaluator using heart rate variability index**
Auswerter für psychische Symptome und psychische Erkrankungen mittels Herzfrequenzvariabilitätsindex
Évaluateur de risque d'apparition de symptômes et de troubles psychiatrique utilisant un indice de variabilité du rythme cardiaque

(43) Date of publication of application: 22.04.2015
(73) Proprietor: GM3 Co., Ltd., Tokyo (JP); Shinba, Toshikazu, Tokyo (JP); Tokuhara, Koujirou, Tokyo (JP)
(72) Inventor: Shinba, Toshikazu, Tokyo (JP)
(74) Representative: Novagraaf Technologies

(56) References cited:
- US-A1- 2004 111 036
- US-A1- 2007 100 246
- US-A1- 2007 173 901
- Kevin Ka-Shing Chan ET AL: "Random Number Generation Deficit in Early Schizophrenia", PERCEPTUAL AND MOTOR SKILLS., vol. 112, no. 1, 1 February 2011 (2011-02-01), pages 91-103, XP055695172, US ISSN: 0031-5125, DOI: 10.2466/02.15.19.22.PMS.112.1.91-103
- Toshikazu Shinba ET AL: "Decrease in heart rate variability response to task is related to anxiety and depressiveness in normal subjects : Heart rate variability responsiveness", PSYCHIATRY AND CLINICAL NEUROSCIENCES, vol. 62, no. 5, 29 September 2008 (2008-09-29), pages 603-609, XP055724891, AU ISSN: 1323-1316, DOI: 10.1111/j.1440-1819.2008.01855.x

## Description

### Technical Field

The present invention relates to a psychiatric symptom and psychiatric disorder onset risk evaluator using a heart rate variability index.
In the text below, references in brackets ([]) refer to the list of references provided at the end of the text.

### Background Art

The beating rate of a heart is always changed. In other words, the interval between heartbeats is extended and shortened in a repetitive manner. This is the heart rate variability, and the change thereof is subjected to control of the autonomic nervous system. The autonomic nervous system is composed of a sympathetic nervous system which arouses and strains the mind and body, and a parasympathetic nervous system which sedates and rests the same. Various physiological and pharmacological researches beginning at the research by Akselrod et al. (American Journal of Physiology, 249: H867-875, 1985 [1]) have found that the component of 0.04 to 0.15 hertz (Low Frequency: LF) of the heart rate variability reflects both activities of the sympathetic nervous system and the parasympathetic nervous system, while the component of 0.15 to 0.4 hertz (High Frequency: HF) thereof reflects the activity of the parasympathetic nervous system. In addition, using the ratio of LF/HF as an index of the sympathetic nervous system activity is practiced by a number of researchers.

These indices are standardized by the European Society of Cardiology, and the like, and are used as a guideline (Malik, M. Heart rate variability: Standards of measurement, physiological interpretation, and clinical use. Circulation 93,1043-1065, 1996 [2]), being utilized for pathology analysis of heart diseases.

In recent years, various attempts to utilize the heart rate variability for evaluation of the mental status have been made, and various methods for accurately and conveniently measuring the heart rate variability have been proposed. For example, in Japanese Patent Publication No. 4568825 [3], in consideration of the fact that the parasympathetic nervous system component of a heart rate variability is closely related to the breathing rhythm, a method for calculating the index of the parasympathetic nervous system activity on the basis of the length of one breath is proposed.

In Japanese Patent Publication No. 4487015 [4], in consideration of the fact that the LF and HF may greatly vary depending upon the individual difference or the difference produced in repetitive recording, it is proposed that a coefficient of variation of RR interval (CVRR) should be incorporated in the mathematical expression to indicate the sympathetic nervous system activity and the parasympathetic nervous system activity with new indices, i.e., (CVRR × LF/(LF + HF)) and (CVRR × HF/(LF + HF)), respectively, and in Japanese Unexamined Patent Publication No. 2008-229223 [5], a short-time Fourier method which uses heartbeat data obtained through as small as 100 beats for analysis is proposed.

In addition, a method for accurately analyzing the mental state through the analysis of the combination with or correlation to another index, such as a brain wave or a blood pressure, is also proposed. For example, in Japanese Patent Publication No. 4410234 [6] and Japanese Unexamined Patent Publication No. 2009-207763 [7], a method is disclosed which analyzes the correlation by determining the pattern of breathing, the rhythm of blood pressure change, the coefficient of R-wave amplitude in an electrocardiograph, and the like in addition to the heart rate variability.

However, while these methods disclosed in the above-mentioned patent documents contribute to the progress of the measuring or analyzing method, these patent documents have no specific disclosure about how to utilize the invention for analysis or diagnosis of the change in mental status or pathology of a psychiatric disorder. Thus, there has been no preferred method for implementing utilization of a heart rate variability index in analysis, diagnosis, or prevention of the change in pathology of a psychiatric disorder, however, there are available the following patent documents which intend implementation thereof, and many of them focus on evaluation of the stress state.

The change in heart rate variability in the stress state can be a change which is a healthy reaction per se, and therefore, the change is not the same as that related to a psychiatric disorder or the risk of onset thereof, however, there are no prior art documents which mention this point.

The invention disclosed in Japanese Patent Publication No. 4637963 [8] uses heart rate variability indices before and after deep breathing and from the difference, checks for health state. It is a publicly known fact that the rhythm of breathing can have an influence on the heart rate variability. The heart rate variability index for the parasympathetic nervous system depends on the rhythm of breathing. The health checking method using the rhythm of breathing is meaningful, however, it is supposed that the method perhaps focuses on the reaction persistence of the parasympathetic nervous system to the breathing. Also about depression, the document states that "it can be simply and instantaneously made clear that there is a high possibility of the subject having depression", however, there have been no clinical observations proving that utilization of the heart rate variability due to the breathing load is useful for identification of depression. It is supposed that, for evaluation of the psychiatric symptom and the depression, a mental load should be imposed on the subject.

Further, the invention disclosed in Japanese Patent Publication No. 4238321 [9] imposes a mental arithmetic stress on the subject, and from the change in heart rate variability power value and frequency, verifies the existence of a stress. However, in the patent document disclosing this invention, it is stated that "an average power value of 0.2 Hz or under is calculated", but if an average is determined for this frequency band, the average involves both of the sympathetic nervous system component and the parasympathetic nervous system component, thereby the autonomic nervous system evaluation cannot be accurately performed, which is a problem.

Further, for the invention disclosed in Japanese Patent Publication No. 4238321 [9], it is stated that "when the average power frequency is increased to or over a prescribed value with respect to the criterion value, it is judged that the subject is feeling an emotional stress", however, there is no discussions about a fact that responding to a stressor is different from falling in a pathologic stress condition, which is a problem. In other words, it is often a healthy activity that the autonomic nervous system reacts to a mental load, while, in a pathologic stress condition, such reaction is not caused, is excessive, is delayed or disordered, but the invention is lacking consideration about this point.

Further, with the invention disclosed in Japanese Patent Publication No. 3250474 [10], the heart rate and the indices of the sympathetic and parasympathetic nervous system activities are recorded in the resting state and the task performing state, the data being expressed as a distribution graph, and determines that, if the data for one state is overlapped on that for the other in many places, the subject has a stress.

However, this method gives indices of the sympathetic and parasympathetic nervous system activities on the basis of the dispersion of change in heart rate, such indices involving the activities of both sympathetic nervous system and parasympathetic nervous system, however, both of these activities have meanings in the contradictory directions, i.e., positive and negative ones concerning the mental status, thereby utilization of the indices having a mixture of those presents a problem. In addition, utilizing the unitary evaluation method, which is based on only the overlapping of distribution data in the resting state and that in the task performing state, for evaluation of a complicated mental status is difficult, as with the inventions mentioned above.

The invention disclosed in US Patent Publication No. 2007/173901 [11] discloses a method for detecting and/or treating depression using one or more depression parameters such as heart rate variability measurement and/or blood-borne factors. To this aim LF, HF and LF/HF ratio are measured in a steady state and the LF/HF ratio is compared to a threshold value.

### Summary of Invention

### Technical Problem

The heart rate fluctuation and other various autonomic nervous system activities are carried out under the control of the controlling center in the brain stem, however, it is known that they are heavily influenced by the mental status. The modern society gives much stress, and the incidence rate of the psychiatric disorder, such as depression, the number of employees on administrative leave and that of suicides are being increased. However, the diagnosis of the psychiatric symptom is subjective, and as described above, the early treatment and prevention using an objective index have not yet been put into practical use.

The present invention has been made in view of such medical and societal situations in which development of an objective evaluation method has been demanded. A psychiatric symptom and psychiatric disorder onset risk evaluation method (not covered by the appended claims) is disclosed herein and based on a psychiatric symptom and psychiatric disorder onset risk evaluator, and it is an object of the present invention to provide the psychiatric symptom and psychiatric disorder onset risk evaluator using a heart rate variability index that are useful for biologically evaluating the psychiatric symptom through the analysis of the autonomic nervous system activity on the basis of a heart rate variability measurement, and effective for understanding the psychiatric symptom and checking for psychiatric disorder onset risk.

### Solution to Problem

The inventor of the present invention has considered that the problems of the prior art in the field of heart rate variability analysis are attributable to the fact that there is an individual difference in heart rate variability index value, and that only the measurement in the steady state, such as the resting state, is used for analysis, and found that, in order to analyze an important pathology of psychiatric disorder of the disability of mind to respond in different situations, it is necessary to examine the responsiveness to situation and the ability to control, having completed the present invention.

The method (not covered by the appended claims) for utilizing a heart rate variability index for diagnosis of a psychiatric disorder or detection of the risk of onset thereof is disclosed herein, including:
the task presentation and frequency analysis step at which frequency analysis is performed on a measured heart rate and a measured heartbeat interval of a subject for determining a high-frequency index, a low-frequency index and a ratio therebetween;
the sympathetic nervous system and parasympathetic nervous system index calculation step at which a sympathetic nervous system index and a parasympathetic nervous system index are calculated from the result of the frequency analysis; and
the index comparison evaluation step at which the heart rate, sympathetic nervous system index and parasympathetic nervous system index calculated are compared, for evaluation, with threshold values which have been determined from the heart rate, the sympathetic nervous system index, and the parasympathetic nervous system index of a healthy subject that have been previously calculated and stored,
the high-frequency index, the low-frequency index, and the ratio therebetween at the frequency analysis step being those which have been obtained through the measurement and frequency analysis in three states, i.e., the resting state, the task performing state, and the resting state after task performance of the subject, respectively, and
the threshold values being set so as to reflect the psychiatric disorder and the risk of onset thereof in a multilateral manner for every index in the respective states.

Preferably, as the task to be imposed on the subject at the frequency analysis step, a task with which the individual difference in achievement level is small is used, and as the task with which the individual difference in achievement level is small, random number generation is used.

The invention solves the above mentioned problems by furnishing a psychiatric symptom and psychiatric disorder onset risk evaluator using a heart rate variability index, including:
a heart rate variability analyzer and a random number generation analyzer,
wherein said random number generation analyzer is configured to include a random number input unit, a random number score calculation unit, and a display unit (8), which presents a random number generation task to a subject, and
said heart rate variability analyzer is configured to include:
   - a heart rate measuring instrument, which gives a heart rate value for said subject in the resting state before task presentation, the task performing state, and the resting state after task performance of said subject, respectively,
   - a heartbeat interval measuring instrument, which gives a heartbeat interval for said subject in the resting state before task presentation, the task performing state, and the resting state after task performance of said subject, respectively,
   - a frequency analyzer, which performs frequency analysis of a heart rate value and a heartbeat interval of said subject that have been measured in the resting state before task presentation, the task performing state, and the resting state after task performance of said subject, respectively, for calculating a high-frequency index, a low-frequency index, and a ratio therebetween,
   - a sympathetic/parasympathetic nervous system index analyzer, which calculates a sympathetic nervous system index and a parasympathetic nervous system index from the result of the frequency analysis, and
   - a psychiatric symptom/psychotic disorder onset risk calculator which includes a storage unit storing threshold values which have been previously set on the basis of a high-frequency index, a low-frequency index and a ratio therebetween which have been obtained from frequency analysis of a heart rate and a heartbeat interval of a healthy subject that have been measured in the resting state before task presentation, the task performing state, and the resting state after task performance, respectively, and a comparison unit comparing the relevant threshold values with the high-frequency index, the low-frequency index and the ratio therebetween of said subject,
upon, as a result of the comparison in said comparison unit, it having been shown that a certain one of said threshold values having been exceeded, said subject being determined to have a high risk of contracting a psychiatric symptom or a psychiatric disorde

The invention is only defined by an apparatus according to independent claim 1, while the disclosed method is not covered by the claims.

### Advantageous Effects of Invention

The present invention is as described above, and it reflects the fact that the sympathetic nervous system index and the parasympathetic nervous system index in the task performing state and the resting state after task performance are different from the index in the resting state before task performance, and determines and analyzes the high-frequency index, the low-frequency index, and the ratio therebetween in the resting state, the task performing state, and the resting state after task performance of the subject, respectively, at the frequency analysis step, thereby the relevant data being extremely effective as data for determining whether there is a prescribed psychiatric symptom and evaluating the psychiatric disorder onset risk, and therefore allowing determination of whether there is a psychiatric symptom and evaluation of the psychiatric disorder onset risk to be performed properly.

In addition, as the task to be imposed on the subject at the frequency analysis step, a task with which a difference due to the capability or experience of the subject is not easily produced, and the individual difference in achievement level is small is used, and for this, random number generation is used for example, thereby the data for the high-frequency index, the low-frequency index, and the ratio therebetween in the resting state, the task performing state, and the resting state after task performance of the subject that is used for evaluating the pathological condition being effective data which will not be influenced by the individual difference in task achievement level.

### Brief Description of Drawings

- Figure 1 is a chart illustrating the flow of operation by the psychiatric symptom and psychiatric disorder onset risk evaluation method disclosed herein;
- Figure 2 is a diagram illustrating the configuration of a psychiatric symptom and psychiatric disorder onset risk evaluator in accordance with the present invention; The reference sign A denotes a heart rate variability analyzer; B a random number generation analyzer; 1 a heart rate measuring instrument; 2 a heartbeat interval measuring instrument; 3 a frequency analyzer; 4 a sympathetic/parasympathetic nervous system index analyzer; 5 a psychiatric symptom/psychiatric disorder onset risk calculator; 6 random number input unit; 7 a random number score calculation unit; and 8 a display unit.
- Figure 3 gives graphs as an example of measurement results and pattern diagrams for a healthy subject illustrating the psychiatric symptom and psychiatric disorder onset risk evaluation method disclosed herein; and
- Figure 4 gives heart rate variability index distribution diagrams illustrating the psychiatric symptom and psychiatric disorder onset risk evaluation method disclosed herein.

The method (not covered by the appended claim) disclosed herein and based on the psychiatric symptom and psychiatric disorder onset risk evaluator in accordance with the present invention, is a method for utilizing a heart rate variability index to make diagnosis of a psychiatric disorder or detection of the onset risk, however, it is a method which analyzes the three distinct conditions of anxiety strain, avolition, and fatigue rather than an unarticulated mind and body sickness which is expressed by a word of stress, and which, in order to allow the analysis thereof, utilizes the data obtained in the three states of the resting state before task presentation, the task performing state, and the resting state after task performance.

In other words, the psychiatric symptom and psychiatric disorder onset risk evaluation method using a heart rate variability index includes:
the task presentation and frequency analysis step at which frequency analysis is performed on a measured heart rate and a measured heartbeat interval of a subject for determining a high-frequency index, a low-frequency index and a ratio therebetween;
the sympathetic nervous system and parasympathetic nervous system index calculation step at which a sympathetic nervous system index and a parasympathetic nervous system index are calculated from the result of the frequency analysis; and
the index comparison evaluation step at which the heart rate, sympathetic nervous system index and parasympathetic nervous system index calculated are compared, for evaluation, with threshold values which have been determined from the heart rate, the sympathetic nervous system index, and the parasympathetic nervous system index of a healthy subject that have been previously calculated and stored,
the high-frequency index, the low-frequency index and the ratio therebetween at the frequency analysis step being those which have been obtained through the measurement and frequency analysis in the resting state, the task performing state, and the resting state after task performance of the subject, respectively, and
the threshold values being set so as to reflect the psychiatric symptom and the psychiatric disorder onset risk.

### Description of Embodiments

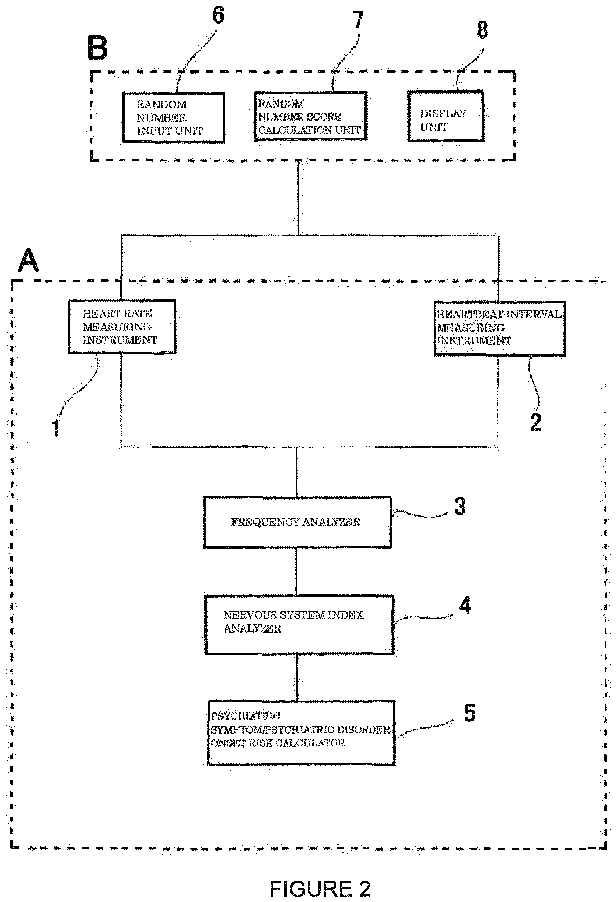

The psychiatric symptom and psychiatric disorder onset risk evaluator using a heart rate variability index in accordance with the present invention is an evaluator for implementing the above-mentioned method, and as shown in Figure 2, the evaluator includes a heart rate variability analyzer A, and a random number generation analyzer B, utilizing random number generation as a task to be imposed on a subject. The heart rate variability analyzer A is configured to include a heart rate measuring instrument 1, a heartbeat interval measuring instrument 2, a frequency analyzer 3, a sympathetic/parasympathetic nervous system index analyzer 4, and a psychiatric symptom/psychiatric disorder onset risk calculator 5. In addition, the random number generation analyzer B is configured to include a random number input unit 6, a random number score calculation unit 7, and a display unit 8.

With the heart rate variability analyzer A, the heart rate measuring instrument 1 and the heartbeat interval measuring instrument 2 give a heart rate value and a heartbeat interval measurement for the subject in the resting state, the task performing state, and the resting state after task performance of the subject, respectively. And, with the frequency analyzer 3, frequency analysis of those measurements is performed for calculating a high-frequency index, a low-frequency index and a ratio therebetween.

The psychiatric symptom/psychiatric disorder onset risk calculator 5 includes a storage unit and a comparison unit, the storage unit storing threshold values which have been previously set as criteria for evaluation. These threshold values are set by imposing random number generation on a healthy subject as a task in the same manner as on the subject, making frequency analysis of the heart rate value and the heartbeat interval measurement which have been given in the resting state before task performance, the task performing state, and the resting state after task performance, respectively, to obtain a high-frequency index, a low-frequency index and a ratio therebetween, and, for each of those, referencing an investigational case. And, with the comparison unit in the psychiatric symptom/psychiatric disorder onset risk calculator 5, such threshold values are compared with the high-frequency index, the low-frequency index and the ratio therebetween of the subject, respectively, which have been sent from the sympathetic/parasympathetic nervous system index analyzer 4, and depending upon whether those indices exceed the relevant threshold value, and to what degree they exceed, whether there is a psychiatric symptom or not, and the psychiatric disorder onset risk are evaluated.

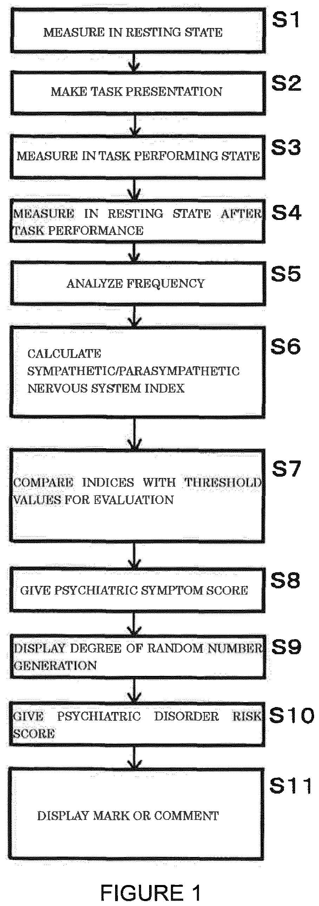

Next, the method (not covered by the appended claim) disclosed herein and based on the psychiatric symptom and psychiatric disorder onset risk evaluator in accordance with the present invention, will be described in detail in the order of step with reference to the flowchart shown in Figure 1.

### Task presentation and frequency analysis step

The task presentation and frequency analysis step is a step (composed of step S1 to S5) which performs frequency analysis on the heart rate measurement and the heartbeat interval measurement for the subject to determine the high-frequency index, the low-frequency index and the ratio therebetween. For measuring the heart rate of the subject, an existing heart rate measuring system may be used. In addition, on the basis of the heartbeat interval obtained from an electrocardiograph or a pulse wave, frequency analysis of the heart rate trend data is performed to determine the high-frequency index (HF), the low-frequency index (LF), and the ratio (LF/HF) between both indices, which have been used on various apparatuses up to now. Determining the HF, LF, and LF/HF values in this way has been conventionally performed, and it is known that the HF index reflects the parasympathetic nervous system activity, the LF index reflecting both of the sympathetic and sympathetic nervous system activities, and the LF/HF index the sympathetic nervous system activity.

However, with the method disclosed herein, the high-frequency index, the low-frequency index, and the ratio therebetween are obtained by making frequency analysis (at step S5) of the data which has been measured in the resting state (at step S1), the task performing state (at step S3), and the resting state after task performance (at step S4) of the subject, respectively. And, the performance task to be imposed on the subject at step S2 is preferably a task with which the individual difference in achievement level is small, in other words, a difference due to the capability or experience of the subject is not easily produced (the task, such as mental arithmetic, which is used by the invention disclosed in Japanese Patent Publication No. 4238321 [9] provides a great individual difference, thereby there being a possibility that effective data may not be obtained), and thus, it is recommended to use, for example, a random number generation task, however, the task is, of course, not limited to this, and any task equivalent thereto will suffice.

For example, the random number generation task should have a content of "saying numbers of one-digit from 0 to 9 in an order as random as possible". And, the subject is instructed to "do his or her best", being away from the initial relax state. The random number generation itself reflects the degree of attention, and the task performing state is evaluated on the randomness score. For calculating the randomness score, the counting bias and interval bias, the random number generation index, etc. can be used (Shinba et al., Perceptual and Motor Skills 91, 1091-1105, 2000). Before the test, checking for anxiety depression state is performed by the self-filling-in type questionnaire sheet method.

### Sympathetic/parasympathetic nervous system index calculation step

The sympathetic/parasympathetic nervous system index calculation step (step S6) is a step at which the sympathetic nervous system index and the parasympathetic nervous system index are calculated from the result of the frequency analysis. As described above, with the method disclosed herein, the data is used which has been obtained in the three states, i.e., the resting state, the task performing state, the resting state after task performance. In other words, the heart rate measurement and the heartbeat interval measurement obtained in each of these three states are frequency-analyzed to determine/calculate the three items, i.e., the heart rate (HR), the sympathetic nervous system activity index (LF/HF), and the parasympathetic nervous system activity index (HF), thereby nine indices in total being obtained to be stored for use in evaluation of the pathology, and the like.

### Index comparison evaluation step

The index comparison evaluation step (step S7) is a step at which the sympathetic nervous system index and the parasympathetic nervous system index, which have been calculated and stored, are compared, for evaluation, with threshold values which have been determined from the sympathetic nervous system index and the parasympathetic nervous system index of a healthy subject that have been previously calculated and stored. The sympathetic nervous system index and the parasympathetic nervous system index of a healthy subject in this case are also obtained in the same manner as those of the subject by making frequency analysis of the heart rate measurement and the heartbeat interval measurement of the healthy subject that have been obtained in the resting state, the task performing state, and the resting state after task performance, respectively. And, the threshold values, which are previously set as comparison criteria, are set so as to reflect the psychiatric symptom and the psychiatric disorder onset risk (varying depending upon the type of psychiatric disorder).

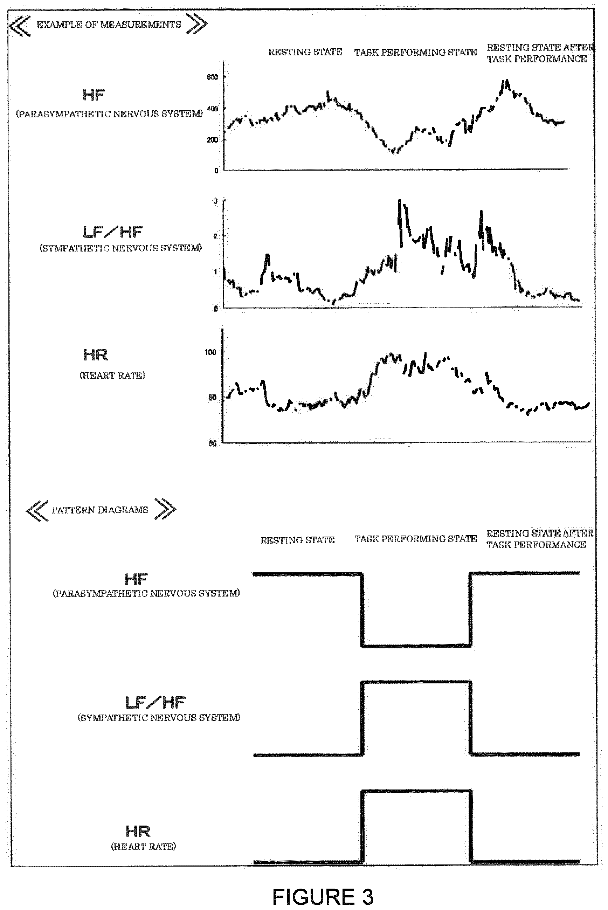

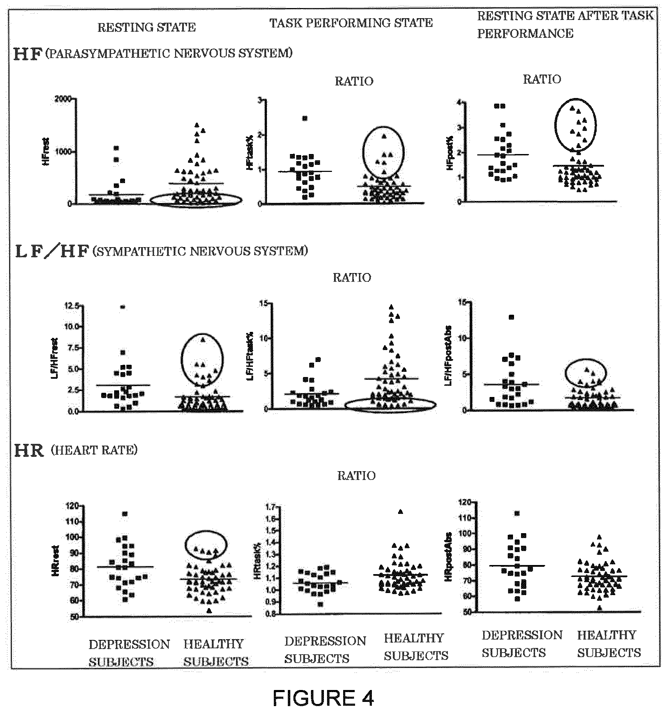

The heart rate variability index of a healthy subject is biased in activity balance to the parasympathetic nervous system side in the initial resting state, and in the task performing state, the parasympathetic nervous system index is lowered, while, in the resting state after task performance, the original condition is restored (refer to the example of measurements and the pattern diagrams in Figure 3). And, if a reaction which is greatly deviated from that in the healthy activity state is recognized, in other words, if the difference from the threshold value exceeds a prescribed value, the condition is detected as an abnormal one. The inventor of the present invention has used the data for fifty healthy subjects and twenty-two depression subjects to obtain a finding which provides a statistically significant difference (Figure 4), and it is supposed that the method disclosed herein has a high credibility in risk evaluation for the psychiatric disorder including depression.

For example, Figure 4 gives an example of utilization of the evaluation method for depression, showing distributions of the data for nine different heart rate variability indices of depressives (at left) and healthy subjects (at right). Among the nine graphs, those to which the word "ratio" is attached use the ratio between indices (rel-rest), which is later described in the paragraph [0038]. The data as a whole exhibits a clear difference between the group of depressives and that of healthy subjects, however, the data distribution in a portion of the graph for the group of healthy subjects that is circled is similar to that in the corresponding portion of the graph for the group of depressives, and is evaluated to provide a possible risk of depression onset. For another type of psychiatric disorder, the method for utilizing the indices is altered for an optimum result.

As described above, the method disclosed herein uses the nine heart rate variability indices in total, i.e., the HF, LF/FH, and heart rate (HR) in the three states, i.e., the resting state (abbreviated to "rest"), the task performing state (abbreviated to "task"), and the resting state after task performance (abbreviated to "post") to evaluate the autonomic nervous system imbalance for performing diagnosis of a stress related psychiatric disorder, such as depression, and clarifying the risk thereof.

The nine different indices can be further utilized as the absolute value (abbreviated to "abs") and the ratio or difference in relation to the index in the resting state (abbreviated to "rel-rest"), or the ratio or difference in relation to the index in the task performing state (abbreviated to "rel-task"). For example, the nine different indices can be used, being expanded to the following eighteen different indices:
1) HFrest-abs,
2) HFtask-abs,
3) HFtask-rel-rest,
4) HFpost-abs,
5) HFpost-rel-rest,
6) HFpost-rel-task,
7) LF/HFrest-abs,
8) LF/HFtask-abs,
9) LF/HFtask-rel-rest,
10) LF/HFpost-abs,
11) LF/HFpost-rel-rest,
12) LF/HFpost-rel-task,
13) HRrest-abs,
14) HRtask-abs,
15) HRtask-rel-rest,
16) HRpost-abs,
17) HRpost-rel-rest, and
18) HRpost-rel-task.

The value in the resting state expresses the state in which the subject is relaxed; the value in the task performing state expresses the reaction upon the stress being imposed; and the value in the resting state after task performance depicts the change through which the nervous system restores the relaxed state, these values being used for evaluation of the anxiety strain state, the avolition, and the fatigue or somatic symptom, respectively. Figure 3 gives one example of using the values, and in this example, from the data obtained by continuously determining the HF value and LF/HF value, and HR value every 2 seconds for approx. 100 seconds in the resting state, for approx. 100 seconds in the random number generation task performing state, and for approx. 100 seconds in the resting state after task performance, average values were calculated for the above-mentioned three respective time periods for use as the indices. In the example given in Figure 3, because the data was analyzed by the maximum entropy method every 2 seconds for 1 minute, the numerical values obtained over 1 minute from 30 seconds after the start of the respective states were averaged.

For example, threshold values of a, b, c, d, e, f, and g (which are given in the expression of HFrest < a, HFtask-rel-rest > b, HFpost-rel-rest > c, LF/HFrest > d, LF/HFtask-rel-rest < e, LF/HFpost-abs > f, and HRrest > g, respectively) are set, and if the index concerned meets the relevant expression, a psychiatric symptom score is given as an observation (at step S8). Such psychiatric symptom scores can be used alone or in combination.
The threshold values are determined from the difference in data between the healthy subject and the subject.

Each of the psychiatric symptom score constituted by a combination of the above-mentioned heart rate variability index scores, the questionnaire sheet score, and the random number generation score, or a combination of these is used as a psychiatric disorder risk score (at step S8 to step S10). If the psychiatric disorder risk score exceeds a certain threshold value, it is determined that there is a high risk that the subject may contract a disease.
The psychiatric symptom score or the psychiatric disorder risk score may be expressed as a mark or a comment for use as a result of measurement (at step S11).

The present invention relates to an apparatus which utilize a heart rate variability index for diagnosis of a psychiatric disorder, such as depression, or detection of the risk of onset thereof, however, the present invention has been made on the basis of the finding of the inventor of the present invention that "the sympathetic nervous system indices and the parasympathetic nervous system indices in the task performing state and the state after task performance are different from the indices in the resting state before task performance", and until now there have been made no approaches from such viewpoint. In case where a conventional heart rate variability index which has not been grounded on such an approach is used for evaluation, only the value obtained in one state, such as the resting state, is mainly used, and therefore, in order to analyze an important pathology of psychiatric disorder of disability to respond to a change, it would become necessary to examine the responsiveness to situation and the ability to control.

The method disclosed herein divides the sympathetic nervous system activity index and the parasympathetic nervous system activity index into the basic value in the resting state, the reaction value in the task performing state, and the recovery value in the resting state after task performance for analysis, and evaluates the pathological condition in the respective three states, while it offers another advantage that the somatic symptoms, such as anxiety strain, avolition, and fatigue, which are a plurality of aspects of the psychiatric disorder, such as depression, anxiety disorder, and stress disorder, can be analyzed in separate and multilateral manners.

### [Reference Signs List]

The reference sign A denotes a heart rate variability analyzer; B a random number generation analyzer; 1 a heart rate measuring instrument; 2 a heartbeat interval measuring instrument; 3 a frequency analyzer; 4 a sympathetic/parasympathetic nervous system index analyzer; 5 a psychiatric symptom/psychiatric disorder onset risk calculator; 6 random number input unit; 7 a random number score calculation unit; and 8 a display unit.

### List of references

1. Akselrod et al. (American Journal of Physiology, 249: H867-875, 1985
2. Malik, M. Heart rate variability: Standards of measurement, physiological interpretation, and clinical use. Circulation 93,1043-1065, 1996
3. Japanese Patent Publication No. 4568825
4. Japanese Patent Publication No. 4487015
5. Japanese Unexamined Patent Publication No. 2008-229223
6. Japanese Patent Publication No. 4410234
7. Japanese Unexamined Patent Publication No. 2009-207763
8. Japanese Patent Publication No. 4637963
9. Japanese Patent Publication No. 4238321
10. Japanese Patent Publication No. 3250474
11. US Patent Publication No. 2007/173901

## Claims

1. A psychiatric symptom and psychiatric disorder onset risk evaluator using a heart rate variability index, comprising :
a heart rate variability analyzer (A) and a random number generation analyzer (B),
wherein said random number generation analyzer (B) is configured to include a random number input unit (6), a random number score calculation unit (7), and a display unit (8), configured to present a random number generation task to a subject, and
said heart rate variability analyzer (A) is configured to include :
- a heart rate measuring instrument (1), configured to give a heart rate value for said subject in the resting state before task presentation, the task performing state, and the resting state after task performance of said subject, respectively,
- a heartbeat interval measuring instrument (2), configured to give a heartbeat interval for said subject in the resting state before task presentation, the task performing state, and the resting state after task performance of said subject, respectively,
- a frequency analyzer (3), configured to perform frequency analysis of a heart rate value and a heartbeat interval of said subject that have been measured in the resting state before task presentation, the task performing state, and the resting state after task performance of said subject, respectively, for calculating a high-frequency index, a low-frequency index, and a ratio therebetween for each state,
- a sympathetic/parasympathetic nervous system index analyzer (4), configured to calculate a sympathetic nervous system index and a parasympathetic nervous system index from the result of the frequency analysis, and
- a psychiatric symptom/psychotic disorder onset risk calculator (5), which includes a storage unit configured to store threshold values which have been previously set on the basis of a high-frequency index, a low-frequency index and a ratio therebetween which have been obtained from frequency analysis of a heart rate and a heartbeat interval of a healthy subject that have been measured in the resting state before task presentation, the task performing state, and the resting state after task performance, respectively, and a comparison unit configured to compare the relevant threshold values with the high-frequency index, the low-frequency index and the ratio therebetween of the subject for each state,
upon, as a result of the comparison in said comparison unit, it having been shown that a certain one of said threshold values having been exceeded, said subject being determined to have a high risk of contracting a psychiatric symptom or a psychiatric disorder.

## Patentansprüche

1. Auswerter für das Risiko des Einsetzens psychischer Symptome und psychischer Erkrankungen mittels Herzfrequenzvariabilitätsindex, umfassend:
einen Herzfrequenzvariabilitätsanalysator (A) und einen Analysator (B) zur Generierung statistischer Zahlen,
wobei der Analysator (B) zur Generierung statistischer Zahlen so ausgestaltet ist, dass er eine Eingabeeinheit (6) für statistische Zahlen, eine Berechnungseinheit (7) eines statistischen Zahlen-Scores und eine Anzeigeeinheit (8) einschließt, die ausgestaltet ist, um einem Subjekt eine Aufgabe zur Generierung von statistischen Zahlen zu präsentieren, und
wobei der Herzfrequenzvariabilitätsanalysator (A) so ausgestaltet ist, dass er einschließt:
- ein Herzfrequenzmessinstrument (1), das ausgestaltet ist, um einen Herzfrequenzwert für das Subjekt in dem Ruhezustand vor der Präsentation der Aufgabe, im Aufgabendurchführungszustand beziehungsweise dem Ruhezustand nach Durchführen der Aufgabe durch das Subjekt zu geben,
- ein Herzschlagintervallmessinstrument (2), das ausgestaltet ist, um ein Herzschlagintervall für das Subjekt in dem Ruhezustand vor der Präsentation der Aufgabe, dem Aufgabendurchführungszustand beziehungsweise dem Ruhezustand nach Durchführen der Aufgabe durch das Subjekt zu geben,
- einen Frequenzanalysator (3), der ausgestaltet ist, um Frequenzanalyse eines Herzfrequenzwerts und eines Herzschlagintervalls des Subjekts durchzuführen, die in dem Ruhezustand vor Präsentation der Aufgabe, dem Aufgabendurchführungszustand beziehungsweise in dem Ruhezustand nach Durchführen der Aufgabe durch das Subjekt gemessen wurden, um einen Hochfrequenzindex, einen Niederfrequenzindex und ein Verhältnis zwischen diesen für jeden Zustand zu berechnen,
- einen Analysator (4) des Index des sympathischen/parasympatischen Nervensystems, der ausgestaltet ist, um einen Index des sympathischen Nervensystems und einen Index des parasympathischen Nervensystems aus dem Ergebnis der Frequenzanalyse zu berechnen, und
- einen Berechner (5) für das Risiko des Einsetzens psychischer Symptome/psychischer Erkrankungen, der eine Speichereinheit, die ausgestaltet ist, um Schwellenwerte zu speichern, die zuvor basierend auf einem Hochfrequenzindex, einem Niederfrequenzindex und einem Verhältnis dazwischen festgelegt wurden, die aus der Frequenzanalyse einer Herzfrequenz und eines Herzschlagintervalls eines gesunden Subjekts erhalten wurden, die in dem Ruhezustand vor der Präsentation der Aufgabe, dem Aufgabendurchführungszustand beziehungsweise dem Ruhezustand nach der Aufgabendurchführung gemessen werden, und eine Vergleichseinheit einschließt, die ausgestaltet ist, um die relevanten Schwellenwerte mit dem Hochfrequenzindex, dem Niederfrequenzindex und dem Verhältnis zwischen diesen von dem Subjekt für jeden Zustand zu vergleichen, wobei als Ergebnis des Vergleichs, für den in der Vergleichseinheit gezeigt worden ist, dass ein bestimmter der Schwellenwerte überschritten worden ist, bestimmt wird, dass das Subjekt ein hohes Risiko aufweist, sich ein psychisches Symptom oder eine psychische Erkrankung zuzuziehen.

## Revendications

1. Evaluateur du risque d'apparition de symptômes et de troubles psychiatriques utilisant un indice de variabilité du rythme cardiaque, comprenant :
un analyseur de variabilité du rythme cardiaque (A) et un analyseur de génération de nombres aléatoires (B),
dans lequel ledit analyseur de génération de nombres aléatoires (B) est configuré pour inclure une unité de saisie de nombres aléatoires (6), une unité de calcul de score de nombres aléatoires (7), et une unité d'affichage (8), configurée pour présenter une tâche de génération de nombres aléatoires à un sujet, et
ledit analyseur de variabilité du rythme cardiaque (A) est configuré pour inclure :
- un instrument de mesure du rythme cardiaque (1), configuré pour donner une valeur de rythme cardiaque pour ledit sujet dans l'état de repos avant la présentation de la tâche, l'état d'exécution de la tâche, et l'état de repos après l'exécution de la tâche dudit sujet, respectivement,
- un instrument de mesure de l'intervalle des battements de cœur (2), configuré pour donner un intervalle des battements de cœur pour ledit sujet dans l'état de repos avant la présentation de la tâche, l'état d'exécution de la tâche, et l'état de repos après l'exécution de la tâche dudit sujet, respectivement,
- un analyseur de fréquence (3), configuré pour effectuer une analyse de fréquence d'une valeur de rythme cardiaque et d'un intervalle de battement de cœur dudit sujet qui ont été mesurés dans l'état de repos avant la présentation de la tâche, l'état d'exécution de la tâche, et l'état de repos après l'exécution de la tâche dudit sujet, respectivement, pour calculer un indice de haute fréquence, un indice de basse fréquence, et un rapport entre eux pour chaque état,
- un analyseur d'indice du système nerveux sympathique / parasympathique (4), configuré pour calculer un indice du système nerveux sympathique et un indice du système nerveux parasympathique à partir du résultat de l'analyse de fréquence, et
- un calculateur du risque d'apparition de symptômes psychiatriques/troubles psychotiques (5), qui comprend une unité de stockage configurée pour stocker des valeurs seuils qui ont été préalablement fixées sur la base d'un indice de haute fréquence, d'un indice de basse fréquence et d'un rapport entre eux qui ont été obtenus à partir de l'analyse de la fréquence d'un rythme cardiaque et d'un intervalle de battement du cœur d'un sujet sain qui ont été mesurés à l'état de repos avant la présentation de la tâche, l'état d'exécution de la tâche et l'état de repos après l'exécution de la tâche, respectivement, et une unité de comparaison configurée pour comparer les valeurs seuils pertinentes avec l'indice de haute fréquence, l'indice de basse fréquence et le rapport entre eux du sujet pour chaque état,
dès lors que, du fait de la comparaison dans ladite unité de comparaison, il a été démontré qu'une certaine desdites valeurs seuils a été dépassée, ledit sujet est déterminé comme présentant un risque élevé de contracter un symptôme psychiatrique ou un trouble psychiatrique.
